**Europäisches Patentamt**

**European Patent Office**

**Office européen des brevets**

(11) Publication number: **0 171 970**
**A2**

(12) # EUROPEAN PATENT APPLICATION

(21) Application number: **85305432.8**

(22) Date of filing: **30.07.85**

(51) Int. Cl.⁴: **C 12 N 5/02**

(30) Priority: **01.08.84 GB 8419562**
**21.12.84 GB 8432385**

(43) Date of publication of application:
**19.02.86 Bulletin 86/8**

(84) Designated Contracting States:
**AT BE CH DE FR GB IT LI LU NL SE**

(71) Applicant: **Albright & Wilson Limited**
**Albright & Wilson House Hagley Road West**
**Oldbury Warley West Midlands, B68 ONN(GB)**

(72) Inventor: **Mavituna, Ferda**
**15 Bodmin Drive**
**Bramhall Stockport Cheshire SK7 2HX(GB)**

(72) Inventor: **Williams, Paul David**
**17 Old Street**
**Broadbottom Derbyshire(GB)**

(72) Inventor: **Wilkinson, Andrew Keith**
**50 Moss Lane**
**Altrincham Cheshire WA15 8HW(GB)**

(74) Representative: **Savidge, Roger Gordon**
**Madgwick et al,**
**c/o Albright & Wilson Limited 1 Knightsbridge Green**
**London SW1X 7QD(GB)**

(54) **Preparation of plant cell suspension cultures.**

(57) Live plant tissue is converted directly into a viable, fine suspension culture by exposure to high shear for a short period as in a homogeniser at 13,000 revs. per minute for 10 seconds, followed by culturing in aqueous nutrient medium.

EP 0 171 970 A2

0171970

The present invention relates to the preparation of suspension cultures of plant cells. In particular it provides a relatively cheap and rapid method for preparing suspension cultures which are suitable for use in tissue culture e.g. the inoculation of open porous or reticular bodies to form a supported callus tissue according to E.P.O 052 001, or for cloning or micropropagation of plants.

Hitherto the preparation of suspension cultures of plant cells has been a slow and laborious process, involving subjecting callus tissue or immobilised plant cells to prolonged low intensity abrasion by continuous gentle, shaking or stirring in nutrient medium for a period of weeks, or even months, accompanied by periodic filtering and reculturing. This procedure has considerable drawbacks when it is desired to initiate suspension cultures on a commercial scale.

Typically a suspension culture suitable for inoculating foam supports takes about six months to establish, and usually only a small proportion of such a culture is capable of invading the foam. In addition to the time and labour there is substantial wastage of nutrients and increased risk of contamination. There is also evidence that prolonged cell culturing, especially in suspension may reduce biochemical potential or cause genetic changes. Throughout this initial period aseptic conditions of storage and handling must be maintained.

The only alternative to the foregoing method, to have been proposed hitherto, is by administering enzymes or other chemicals which are alleged to promote disaggregation of plant cells. Depending on the chemicals selected and the concentration used, this method has been found either ineffective, or causes biochemical alteration of the cells and substantial reduction in viability.

Moreover, because of the tendency for plant cells in suspension to form aggregates by repeated division of a single parent cell and/or by agglomeration of two or more cells or aggregates, plant cell suspensions are difficult to maintain. They rapidly become undesirably coarse, unless frequently filtered and subcultured. The preparation and maintenance of plant cell cultures is therefore a time and labour consuming activity. In order to provide a continuing supply of suitable tissue for commercial biosynthesis of plant secondary metabolites, and or micropropagation and cloning, it is necessary to prepare and maintain a master culture of suspended cells which can be used, for instance, to inoculate the solid substrates or supports which are employed for tissue culture. The finer and more uniform such a suspension can be, the more rapidy and easily can foam supported tissue culture be established.

We have now discovered that when live plant tissue or a coarse suspension culture is subjected to relatively high intensity shear for a relatively short time, as by introducing pieces of callus or other tissue or even whole plants into a homogeniser or commercial food blender for short periods of preferably a few seconds up to a few minutes, it is possible to obtain relatively fine and homogeneous cell suspension cultures of high viability very much more rapidly and cheaply than hitherto.

It is particularly surprising that plant cells can be subjected to such drastic treatment and recover to form useful cultures. Hitherto plant cell cultures have been believed to be highly susceptible to damage by even comparitively moderate shear stress. Thus, for instance air lift fermenters are often preferred to stirred fermenters for plant cell culture. "Progress in Industrial Microbiology" Vol 16 (1982) Edited by M.J. Ball includes a review by W. Fowler (pp 216 to 218) of literature on loss of cell viability in stirred fermenters. For instance a rotary stirrer rotating at 250 RPM has been observed to cause unacceptable loss of viability in a plant suspension culture. There is thus a strong prejudice in the art, against exposing plant cells to higher shear.

Care is normally taken to use only very gentle shaking to convert callus to suspension culture, so that cells are detached from the surface of the callus by gradual abrasion over an extended period of many months, residual coarse pieces being filtered off and discarded. Susprisingly, we have now found that when plant tissue is subjected to homogenisation using impeller rates high enough to comminute the tissue rapidly into small suspended particles (e.g. 1,500 to 15,000 RPM), the resulting suspension remains viable and recovers rapidly provided that the time during which the culture is exposed to high shear is sufficiently short.

Our invention therefore provides a method for the preparation of suspension cultures, which comprises exposing live plant tissue preferably in admixture with a non-phytotoxic liquid medium, to mechanical shearing of sufficient intensity to form a suspension of particles of average size substantially smaller than the original tissue, during a period sufficient to permit substantial comminution of the tissue without irreversible loss of viability of the suspension so formed, and subsequently culturing the comminuted tissue in contact with a nutrient medium.

Typically the shear stress is applied by means of a rotary or, less preferably, a reciprocating or oscillating member, such as a rotating blade moving through the medium. The shear rate is preferably the minimum required to produce the desired degree of disaggregation in the selected time. Where the culture is to be used to inoculate open porous or reticular bodies this minimum degree of disaggregation is determined by the maximum particle size that can readily invade the interstices of the bodies.

The maximum velocity of the shearing member i.e. the tip speed, for a rotating member, is usually above 3 m $S^{-1}$ and preferably between about 5 m sec $^{-1}$ and 45 m sec $^{-1}$ e.g. 5.7 to 43.5 ms $^{-1}$ most preferably 34 to 40 ms $^{-1}$. Where a rotating blade or impeller corresponding to those used in commercial food blenders is used to impart shear we prefer that the speed of rotation should be above 1,500 RPM e.g. between 2,000 and 15,000 RPM, more preferably 12,000 to 14,000 RPM e.g. 12,500 to 13,500 RPM.

We prefer to apply shear by rotation of an impeller characterised by low power number $(N_p)$ and high Reynolds number $(N_{Re})$. E.g. $N_p$ less than 0.2 preferably less than 0.1; $N_{Re}$ greater than $10^4$ preferably greater than $10^5$ e.g. greater than $5 \times 10^5$.

The Shear is preferably applied for a period of from 2 seconds to 10 minutes, more preferably 5 seconds to 5 minutes. However somewhat longer or shorter times and higher or lower speeds may be possible or desirable depending on the hardiness of the particular cells, the toughness and cohesiveness of the tissue, the size and geometry of the homogeniser, the viscosity of the liquid medium and the degree of comminution required.

Generally the conditions will be such that the tissue is substantially reduced to particles less than 3mm and preferably less than 1.5mm diameter. For most purposes e.g. cultures which are to be inoculated into foamed polymeric supports, we prefer the particle size of the product to be substantially all less than 1mm. In particular we prefer at least a major proportion of the biomass to be reduced to less than 0.5mm.

We do not exclude the possibility that for certain cultures the desired degree of comminution may be possible at lower shear rates than those indicated above applied for as long as one or two hours or even longer, without unacceptable loss of viability. In selecting an appropriate time care is preferably taken not to allow the % of viable cells in the culture, and especially of the particles in the desired size range, to fall below 30%. We prefer that the shear be removed before the % viability falls to 50%. Most preferably % viability is maintained above 60%.

After homogenising, the cell suspension is usually cultured in a growth sustaining nutrient medium, e.g. for up to 4 weeks, until a suspension culture of high viability (e.g. higher than 90%) is established.

Surprisingly, in view of the unprecedently severe physical stress involved in subjecting plant tissue to such high shear rates, we have discovered that the resulting suspensions recover rapidly to give a percentage cell viability, especially in the finer particle sizes comparable to, or better than those which can be obtained by conventional methods. The latter, however, require three to four times as long a culturing period. Culturing preferably takes place in gently shaken flasks or an air agitated fermenter. Stirring or other operations entailing prolonged exposure to moderate or high shear are preferably avoided.

It is particularly surprising that we have been able to prepare viable suspension cultures from live plants without the time consuming intermediate step of isolating and culturing callus tissue, which has always been considered essential, hitherto. We have found that whole plants or live portions of plants, suitably cleaned, may be chopped up and placed in a homogeniser or other shearing device to provide a fine, viable, relatively homogeneous, suspension culture.

Preferably live plants or pieces thereof, including stems, leaves, fruit, flowers, roots and/or callus or meristem tissue, are sterilised by exposing the surface of the plant or tissue to a suitable biocide which will destroy any contaminating microorganisms without substantial damage to at least the majority of the plant cells, e.g. sodium hypochlorite solution. The sterilised plant is preferably roughly chopped and mixed with a non-phytotoxic liquid medium. The latter is preferably aqeuous e.g. water or an aqueous nutrient medium. The mixture is then introduced into the homogeniser and subjected to high shear stress to form a suspension of cells or small aggregates.

Preferably, after shearing, the suspension is allowed to settle for a short time, e.g. 1 to 10 minutes, usually 2 to 5 minutes, and the supernatant liquid medium is decanted off the sedimented particles. The liquid contains some cell debris, which may inhibit the growth of the culture if not removed from the viable cells which constitute the sediment.

The latter are preferably shaken gently in aqueous nutrient medium for a sufficient period, e.g. 2 to 6 weeks, to establish whatever quantity of viable biomass is required. Alternatively the cells may be used to inoculate agar plates, either directly or after an intermediate period of suspension culture, in order to develop callus, e.g. for cloning or micropropagation.

The invention may be used to prepare suspension cultures directly from live plants without the need first to establish a callus culture, as is required in conventional methods. Alternatively it may be used to prepare suspension cultures from existing callus cultures, without the prolonged period of reculturing normally required to obtain a suspension of acceptable quality and biomass. Finally our invention may be used to refine a coarse existing suspension culture which contains over- sized particles.

Cultures prepared according to our invention may be maintained as suspension cultures, they may establish callus cultures on nutrient plates e.g. for growing into plants, or they may be immobilised using tissue immobilisation technology as described, for example in the aforesaid EP 0052001, e.g. for use in manufacturing secondary metabolites. The last is particularly preferred. We have observed that when open porous or reticular bodies such polyurethane foam particles or wire bundles are placed in a suspension prepared according to our invention, the cells invade the porous particles more rapidly to provide a higher loading of biomass per particle than with an equivalent suspension culture prepared by conventional methods. The inoculated foam supports may be cultured with aqueous nutrient medium in a bioreactor to provide secondary metabolites.

The invention is illustrated by the following examples:

Example 1

5 gm samples of callus tissue of Capsicum frutescens var. annuum were each mixed with 500 mls Schenk and Hildebrandt nutrient medium in a Waring "BLENDOR", (R.T.M.) homogeniser and homogenised for 10 seconds at minimum speed setting.

After 1 minute settling, the supernatant liquor containing the cell debris was decanted and the residue made up to 500 ml with fresh nutrient, in shake flasks.

The flasks were cultured with gentle shaking and sampled at intervals for 30 days.

Table 1. shows the concentration of biomass in different size ranges.

TABLE 1.

| Time (days) | | Total Biomass | Biomass 3.00 mm | 2.75- 3.00mm | 1.25- 2.75mm | 1.00- 1.25mm | 0.50- 1.00mm | 250 500u | 250u |
|---|---|---|---|---|---|---|---|---|---|
| 0 Debris | Wet Weight g.l$^{-1}$ | 98.467 | - | - | 4.088 | 1.456 | 66.514 | 38.884 | 57.576 |
| | Dry Weight g.l$^{-1}$ | 4.062 | - | - | 0.056 | 0.012 | 1.450 | 0.962 | 2.384 |
| | Viability % | 46 | - | - | - | 6 | 37 | 58 | 0 |
| 0 | Wet Weight g.l$^{-1}$ | 84.058 | - | - | - | 3.495 | 7.180 | 11.778 | 31.404 |
| | Dry Weight g.l$^{-1}$ | 1.475 | - | - | - | 0.042 | 0.195 | 0.295 | 1.633 |
| | Viability % | 39 | - | - | - | - | 41 | 57 | 19 |
| 8 | Wet Weight g.l$^{-1}$ | 129.254 | - | - | 7.900 | 4.336 | 35.928 | 14.261 | 51.390 |
| | Dry Weight g.l$^{-1}$ | 3.391 | - | - | 0.264 | 0.167 | 0.928 | 0.356 | 1.994 |
| | Viability % | 71 | - | - | 70 | 42 | 44 | 91 | 37 |
| 15 | Wet Weight g.l$^{-1}$ | 347.727 | 10.914 | 18.636 | 128.343 | 9.800 | 65.379 | 35.025 | 46.429 |
| | Dry Weight g.l$^{-1}$ | 13.833 | 0.532 | 0.850 | 5.032 | 0.407 | 1.489 | 0.775 | 1.368 |
| | Viability % | 95 | 50 | 81 | 60 | 52 | 85 | 51 | 0 |
| 23 | Wet Weight g.l$^{-1}$ | 457.650 | 86.365 | 56.148 | 161.022 | 19.161 | 73.213 | 77.574 | 28.344 |
| | Dry Weight g.l$^{-1}$ | 17.958 | 3.543 | 2.374 | 5.465 | 0.443 | 1.716 | 1.835 | 0.258 |
| | Viability % | 96 | 98 | 94 | 81 | 87 | 12 | 0 | 0 |
| 30 | Wet Weight g.l$^{-1}$ | 454.494 | 155.218 | 24.923 | 108.882 | 9.265 | 48.523 | 86.823 | 49.069 |
| | Dry Weight g.l$^{-1}$ | 17.733 | 4.818 | 0.823 | 3.176 | 0.241 | 1.076 | 1.835 | 0.312 |
| | Viability % | 94 | 94 | 96 | 98 | 67 | 51 | 32 | 0 |

Table 1: Size distribution of growing homogenised callus cultures

Example 2

Homogenisation of Callus at Various RPM

Approximately 10g samples of callus of known viability were each homogenised in 200ml of medium for 20 seconds at various RPMs. Viability, wet weight and dry weight were then taken measured. Initial dry weight of callus was estimated by extrapolation of the weight loss of a dried callus sample. The results are shown in Table 2.

TABLE 2

| Initial FW g.l$^{-1}$ | Initial DW g.l$^{-1}$ | Initial Viability % | RPM | Final FW g.l$^{-1}$ | Final DW g.l$^{-1}$ | Final Viability % |
|---|---|---|---|---|---|---|
| 57.320 | 2.067 | 97 | 2100 | 56.797 | 1.559 | 95 |
| 48.857 | 1.762 | 98 | 2500 | 53.788 | 1.403 | 80 |
| 34.051 | 1.228 | 96 | 3100 | 49.111 | 1.272 | 61 |
| 64.879 | 2.340 | 94 | 3800 | 63.586 | 2.183 | 63 |

Example 3

800ml of a 14 day old suspension culture of Capsicum frutescens in Schenk and Hildebrand nutrient medium was divided into three portions. 100mls was analysed for viability, pH, fresh weight of biomass, dry weight of biomass and size distribution of particles; a 200ml sample was diluted with 400ml medium and used as a control. The remaining 500ml was homogenised for 5 seconds at the low speed setting of the homogeniser, and 200mls of the suspension was made up with 400ml of fresh nutrient. The control and homogenised suspensions were each divided into 10 x 60 ml. samples in 250ml. shake flasks.

The first flasks from the homogenised batch and the control batch were sampled immediately and the remaining flasks were sampled at intervals up to 30 days. Two flasks from the homogenised batch and one from the control batch became contaminated and were therefore not available for sampling. ·Flasks were sampled for viability, pH, fresh weight, dry weight and size distribution.

## Results

Table 3 shows the results in terms of the concentration in $g.1^{-1}$ of biomass in each size range for the control suspension and Table 5 shows these results for the homogenised suspension.

Dry weight figures for each size range are expressed as percentages of total biomass concentration for control suspension in Table 4 and for homogenised suspension in Table 6.

## TABLE 3.
### Biomass

| Time (days) | | pH | Total Biomass | 3.00 mm | 2.75–3.00mm | 1.25–2.75mm | 1.00–1.25mm | 0.50–1.00mm | 250 500u | 250u |
|---|---|---|---|---|---|---|---|---|---|---|
| 0 | Wet Weight g.l$^{-1}$ | 5.6 | 56.000 | 1.429 | 0.655 | 1.280 | 0.942 | 0.563 | 0.162 | 0.002 |
| | Dry Weight g.l$^{-1}$ | | 2.230 | 0.061 | 0.023 | 0.048 | 0.028 | 0.018 | 0.005 | 0.016 |
| | Viability % | | 78 | | | | | | | |
| 4 | Wet Weight g.l$^{-1}$ | 5.3 | 70.550 | 1.745 | 0.542 | 0.918 | 0.236 | 0.295 | 0.147 | 0.001 |
| | Dry Weight g.l$^{-1}$ | | 2.710 | 0.063 | 0.016 | 0.032 | 0.004 | 0.013 | 0.004 | 0.017 |
| | Viability % | | 95 | | | | | | | |
| 8 | Wet Weight g.l$^{-1}$ | 6.9 | 114.089 | 1.981 | 0.557 | 2.036 | 0.708 | 1.582 | 0.180 | 0.141 |
| | Dry Weight g.l$^{-1}$ | | 4.300 | 0.079 | 0.021 | 0.071 | 0.040 | 0.027 | 0.009 | 0.024 |
| | Viability % | | 98 | | | | | | | |
| 11 | Wet Weight g.l$^{-1}$ | 6.8 | 190.325 | 4.919 | 1.110 | 2.202 | 0.576 | 0.844 | 0.313 | 0.122 |
| | Dry Weight g.l$^{-1}$ | | 7.402 | 0.199 | 0.037 | 0.082 | 0.025 | 0.022 | 0.006 | 0.022 |
| | Viability % | | 97 | | | | | | | |
| 16 | Wet Weight g.l$^{-1}$ | 6.3 | 244.144 | 7.986 | 0.767 | 2.956 | 0.749 | 0.867 | 0.194 | 0.396 |
| | Dry Weight g.l$^{-1}$ | | 9.429 | 0.303 | 0.031 | 0.107 | 0.031 | 0.030 | 0.007 | 0.027 |
| | Viability % | | 98 | | | | | | | |
| 22 | Wet Weight g.l$^{-1}$ | 6.2 | 348.161 | 11.976 | 1.688 | 4.990 | 1.852 | 1.240 | 0.389 | 0.843 |
| | Dry Weight g.l$^{-1}$ | | 17.386 | 0.422 | 0.062 | 0.170 | 0.067 | 0.142 | 0.011 | 0.045 |
| | Viability % | | 99 | | | | | | | |
| 24 | Wet Weight g.l$^{-1}$ | 6.6 | 381.831 | 13.374 | 1.230 | 4.886 | 1.580 | 1.791 | 0.343 | 1.614 |
| | Dry Weight g.l$^{-1}$ | | 14.431 | 0.490 | 0.047 | 0.191 | 0.069 | 0.070 | 0.016 | 0.054 |
| | Viability % | | 99 | | | | | | | |
| 28 | Wet Weight g.l$^{-1}$ | 6.5 | 341.102 | 11.286 | 1.137 | 3.263 | 2.990 | 0.977 | 0.260 | 0.547 |
| | Dry Weight g.l$^{-1}$ | | 13.573 | 0.419 | 0.047 | 0.136 | 0.113 | 0.042 | 0.014 | 0.049 |
| | Viability % | | 71 | | | | | | | |
| 30 | Wet Weight g.l$^{-1}$ | 6.6 | 410.602 | 11.575 | 2.251 | 3.617 | 2.054 | 1.035 | 0.294 | 1.347 |
| | Dry Weight g.l$^{-1}$ | | 12.393 | 0.324 | 0.070 | 0.109 | 0.061 | 0.042 | 0.012 | 0.051 |
| | Viability % | | 68 | | | | | | | |

Table 4 Size distribution of growing control suspension culture.

0171970

### TABLE 4

| Time (days) | Biomass | | | | | | |
|---|---|---|---|---|---|---|---|
| | 3.00 mm | 2.75-3.00mm | 1.25-2.75mm | 1.00-1.25mm | 0.05-1.00mm | 250-500u | 250u |
| 0 | 30.6 | 11.6 | 24.1 | 14.2 | 9.0 | 2.5 | 8.0 |
| 4 | 42.3 | 10.7 | 21.5 | 2.7 | 8.7 | 2.7 | 11.4 |
| 8 | 29.1 | 7.7 | 26.2 | 14.8 | 10.0 | 3.3 | 8.9 |
| 11 | 50.6 | 9.4 | 20.9 | 6.4 | 5.6 | 1.5 | 5.6 |
| 16 | 56.5 | 5.8 | 20.0 | 5.8 | 5.6 | 1.3 | 5.0 |
| 22 | 45.9 | 6.7 | 18.5 | 7.3 | 15.4 | 1.2 | 4.9 |
| 24 | 52.3 | 5.0 | 20.4 | 7.4 | 7.5 | 1.6 | 5.8 |
| 28 | 51.1 | 5.7 | 16.6 | 13.8 | 5.1 | 1.7 | 6.0 |
| 30 | 48.4 | 10.5 | 16.4 | 9.0 | 6.3 | 1.8 | 7.6 |

## TABLE 5

| Time (days) | | pH | Total Biomass | Biomass | | | | | | |
|---|---|---|---|---|---|---|---|---|---|---|
| | | | | 3.00 mm | 2.75-3.00mm | 1.25-2.75mm | 1.00-1.25mm | 0.50-1.00mm | 250 500u | 250u |
| 0 | Wet Weight g.l$^{-1}$ | 5.6 | 27.460 | - | - | - | 0.562 | 1.143 | 0.382 | 0.062 |
| | Dry Weight g.l$^{-1}$ | | 1.079 | - | - | - | 0.021 | 0.047 | 0.014 | 0.024 |
| | Viability % | | 64 | | | | | | | |
| 4 | Wet Weight g.l$^{-1}$ | 5.7 | 25.850 | - | - | 0.127 | 0.134 | 0.013 | 0.208 | 0.069 |
| | Dry Weight g.l$^{-1}$ | | 1.635 | - | - | 0.004 | 0.008 | 0.038 | 0.014 | 0.034 |
| | Viability % | | 80 | | | | | | | |
| 8 | Wet Weight g.l$^{-1}$ | 6.5 | 43.800 | - | - | 0.304 | 0.492 | 1.118 | 0.417 | 0.297 |
| | Dry Weight g.l$^{-1}$ | | 2.115 | - | - | 0.013 | 0.019 | 0.045 | 0.015 | 0.034 |
| | Viability % | | 83 | | | | | | | |
| 11 | Wet Weight g.l$^{-1}$ | 6.3 | 88.230 | - | 0.335 | 0.893 | 1.253 | 1.754 | 0.362 | 0.182 |
| | Dry Weight g.l$^{-1}$ | | 3.474 | - | 0.002 | 0.038 | 0.051 | 0.057 | 0.008 | 0.032 |
| | Viability % | | 90 | | | | | | | |
| 16 | Wet Weight g.l$^{-1}$ | 6.3 | 190.706 | 0.438 | 0.394 | 4.497 | 1.470 | 1.296 | 0.353 | 1.152 |
| | Dry Weight g.l$^{-1}$ | | 7.760 | 0.016 | 0.019 | 0.198 | 0.065 | 0.055 | 0.013 | 0.020 |
| | Viability % | | 96 | | | | | | | |
| 22 | Wet Weight g.l$^{-1}$ | 6.6 | 289.735 | 1.104 | 1.961 | 7.709 | 2.968 | 1.959 | 0.360 | 0.453 |
| | Dry Weight g.l$^{-1}$ | | 13.695 | 0.055 | 0.099 | 0.351 | 0.138 | 0.080 | 0.017 | 0.040 |
| | Viability % | | 99 | | | | | | | |
| 24 | Wet Weight g.l$^{-1}$ | 6.6 | 322.136 | 3.478 | 3.448 | 9.076 | 4.621 | 1.926 | 0.394 | 0.616 |
| | Dry Weight g.l$^{-1}$ | | 13.682 | 0.137 | 0.139 | 0.379 | 0.193 | 0.081 | 0.017 | 0.053 |
| | Viability % | | 98 | | | | | | | |
| 28 | Wet Weight g.l$^{-1}$ | 6.6 | 338.646 | 2.910 | 1.467 | 7.216 | 3.748 | 0.201 | 0.201 | 0.299 |
| | Dry Weight g.l$^{-1}$ | | 14.423 | 0.112 | 0.065 | 0.301 | 0.155 | 0.066 | 0.012 | 0.039 |
| | Viability % | | 58 | | | | | | | |

0171970

TABLE 6

| Time (days) | Biomass | | | | | | |
|---|---|---|---|---|---|---|---|
| | 3.00 mm | 2.75- 3.00mm | 1.25- 2.75mm | 1.00- 1.25mm | 0.05- 1.00mm | 250- 500u | 250u |
| 0 | - | - | - | 19.8 | 44.3 | 13.3 | 22.6 |
| 4 | - | - | 4.0 | 8.2 | 38.8 | 14.3 | 34.7 |
| 8 | - | - | 10.3 | 15.1 | 35.7 | 11.9 | 27.0 |
| 11 | - | 1.1 | 20.2 | 27.2 | 30.3 | 4.2 | 17.0 |
| 16 | 4.1 | 4.9 | 51.3 | 16.8 | 14.2 | 3.4 | 5.2 |
| 22 | 7.0 | 12.7 | 45.0 | 17.7 | 10.3 | 2.2 | 5.1 |
| 25 | 13.7 | 13.9 | 37.9 | 19.3 | 8.1 | 1.8 | 5.3 |
| 28 | 14.9 | 8.7 | 40.1 | 20.7 | 8.8 | 1.6 | 5.2 |

Example 4

Carrots were peeled and cut into 4 cm slices, the slices were
sterilised for 25 minutes in 30% aqueous sodium hypochlorite
solution, the outer portion was sliced off and the remainder
homogenised in 500ml. sterile nutrient solution for 10 seconds. The
sample was allowed to settle for 15 minutes. The supernatant liquid
was poured off and discarded. The residue was washed with distilled
water, made up to 500ml. with nutrient medium and divided into
10 x 50ml. samples in 250ml. shake flasks. A viable suspension
culture was formed. It was noted that a characteristic orange
colour, indicative of carotenoid synthesis, continued to be produced
in the culture. In contrast no such colouration is produced in
conventional suspension cultures of carrot cells. This suggests
that suspension cultures produced according to the invention retain
some potential to synthesise secondary metabolites which is often
lost or suppressed in conventional suspension cultures.

Examples 5 to 7

Example 4 was repeated using whole chilli peppers (Example 5) leaves
(Example 6) and 3 cm. lengths of shoot (Example 7) of Capsicum
frutescens var. annuum. In each case a viable suspension culture
was formed.

Example 8

140gm of callus was homogenised by the method of exaple 1, made up
to 550ml. with nutrient medium and divided into 11 x 50ml. samples.
500mls. of medium inoculated with 50ml. of a 24 day old suspension
culture derived from the same cell line was similarly divided into
11 samples as a control. Each sample was placed in a 250ml flask
containing 5 wire reinforced $1cm^3$ polyurethane foam particles
having 30 pores per inch. One flask from each of the homogenised
and control series was analysed immediately and the remainder
sampled at intervals up to 27 days. Foam particles were removed and
analysed for fresh weight, dry weight, volume and viability.

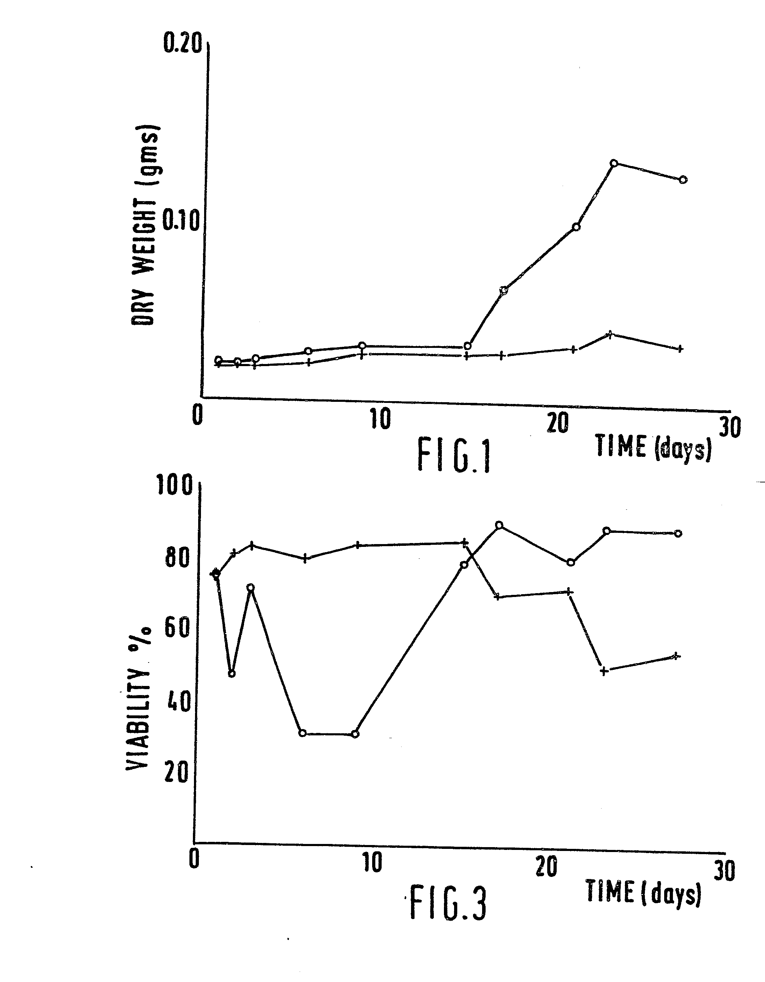

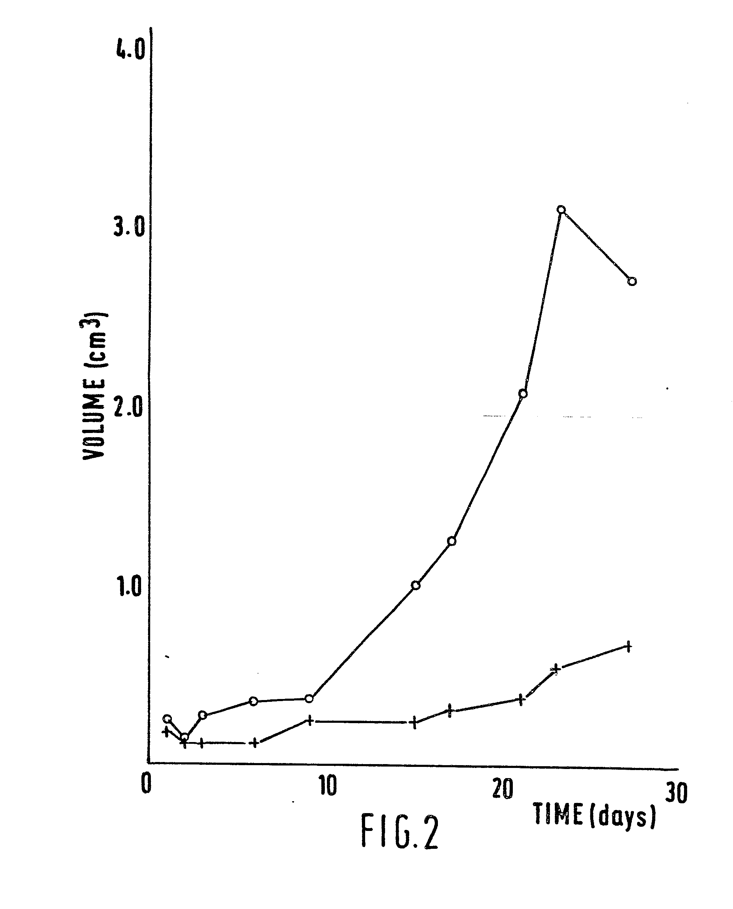

The results are shown in figures 1,2 and 3. Figure 1 is a graph of
the dry weight of the particles, including any immobilised biomass,
against time in days. Figure 2 is a graph of the volume of the
particles, including any outgrowth of biomass, against time in days.
Figure 3 is a graph of the % viability of the cells against time in
days. In each case crosses represent the control and circles the
homogenised system of the present invention.

It will be seen that % viability of the cells in the homogenised
product fell sharply after about twenty four hours to reach a
minimum about five days after shearing. After 10 days the viability
recovered strongly to give a value after 24 days nearly double that
of the control. The dry weight of biomass entrapped by the foam in
the homogenised system also rises sharply, after an induction period
of about 15 days, to about three and a half to four times the reight
in the control culture. Thus the use of the present invention
increased the maximum total number of viable cells per foam by a
factor of about six, compared with conventional methods, in only
half the time from the initiation of the suspension culture.


## Example 9

### Method

Callus of good viability, (better than 95%) at a concentration of
60g Callus in 1l of medium, was homogenised at various speeds and
for various durations. At the end of the homogenisation period the
size range of the remaining biomass, and the viability within each
size range, was determined.


### Results

Initial results of fresh weight, dry weight and viability for each
size range are shown in Tables 7 to 11. Results are further
expressed in terms of the percentage in each size range of total
biomass, the dry weight in each size range of viable biomass and

- 16 -

again as the percentage in each size range of the viable biomass. To **0171970** show the amount of biomass lost completely during the homogenisation process Table 12 shows the viable biomass remaining after homogenisation as a percentage of the intial viable biomass.

## Discussion

In all cases the biomass size was considerably reduced although the profile of size ranges varied. Reaggregation of the biomass began to take place approx. 8 days after homogenisation. The establishment of a fine suspension culture after this period was dependent on the presence of a sufficient biomass concentration of less than 0.5mm. The 13,000 RPM 10 sec experiment gave the most favourable size range profile in these terms but all experiments gave an acceptable profile. However, after homogenisation a proportion of the biomass in each size range is killed and from Table 8 it can be seen that at 335 RPM for 24h all biomass below 1.25mm is killed. The experiments conducted at 3,100 RPM, 1h and 2,100 RPM, 2h both gave the majority of viable biomass in the 0.5-1.0mm size range but both had sufficient biomass below this size to give a slow regeneration to a fine suspension culture. At 3,100 RPM, 17h the large loss of biomass shown in Table 6 means that although the size range of this biomss is good, its poor viability leaves insufficient cells for regrowth. The size range of the 13,000, 10 sec homogenate is good and the proportion of this material which is viable is sufficient to give efficient regeneration.

## Conclusions

The high shear, low duration regime (13,000 RPM, 10 sec) gave by far the best homogenate for growth to suspension culture. The 3,100 RPM, 1h and 2,100 RPM, 2h systems both gave a suspension culture, but with a large amount of reaggregated clumps of cells. 3,100 RPM for 17 h and 335 RPM for 24 h both failed to provide sufficient viable biomass of low particle size to regenerate an acceptably fine suspension culture.

In all the foregoing experiments viability was assessed by the method of Widholme using fluoresceine diacetate.

In all the foregoing experiments, unless stated to the contrary, the homogeniser was run at the the low speed setting. At this setting the impeller rotated at 13,000 RPM (tip speed 37.44 m $S^{-1}$). The power number was 0.0667 and Reynolds number 6.6 x $10^5$.

These were derived from:

$$N_{Re} = \frac{n\ d_i{}^2 \varrho}{m} \ ;$$

$$N_p = \frac{p}{n^3 d_i{}^5 \varrho} \ ;$$

where   $n$   = revs. per sec ;

$d_i$   = impeller diameter ;

$\varrho$   = density ;

$m$   = viscosity ;

$P$   = power imput ;

$N_{Re}$ = Reynolds number ;

$N_p$   = power number ;

- 18 -

0171970

TABLE 7

Homogenate after 10 sec, at 13,000 RPM

| Size range (mm) | 3.0 | 0.75 3.00 | 1.25 2.75 | 1.00 1.25 | 0.50- 1.00 | 0.25- 0.50 | 0.25 |
|---|---|---|---|---|---|---|---|
| Fresh Weight (g) | - | - | - | 3.495 | 7.180 | 11.778 | 31.404 |
| Dry Weight (g) | - | - | - | 0.042 | 0.195 | 0.295 | 1.633 |
| Viability (%) | - | - | - | not.known | 41 | 57 | 19 |
| % Total Dry Weight | - | - | - | 1.900 | 9.000 | 13.600 | 75.400 |
| Viable Dry Weight | - | - | - | not known | 0.080 | 0.168 | 0.310 |
| % Viable Total Weight | - | - | - | not known | 14.300 | 30.100 | 55.600 |

TABLE 8

Homogenate after 24hr, at 335 RPM

| Size range (mm) | 3.0 | 0.75 3.00 | 1.25 2.75 | 1.00 1.25 | 0.50- 1.00 | 0.25- 0.50 | 0.25 |
|---|---|---|---|---|---|---|---|
| Fresh Weight (g) | - | 1.891 | 1.303 | 13.152 | 19.009 | 4.061 | 10.846 |
| Dry Weight (g) | - | 0.051 | 0.035 | 0.414 | 0.448 | 0.116 | 0.772 |
| Viability (%) | - | 65.000 | 8.300 | 0 | 0 | 0 | 0 |
| % Total Dry Weight | - | 2.800 | 1.900 | 22.600 | 24.40 | 6.300 | 42.00 |
| Viable Dry Weight | - | 0.033 | 0.003 | 0 | 0 | 0 | 0 |
| % Viable Total Weight | - | 91.70 | 8.300 | 0 | 0 | 0 | 0 |

## TABLE 9

0171970

Homogenate after 17 h, at 3100 RPM

| Size range (mm) | 3.0 | 0.75-3.00 | 1.25-2.75 | 1.00-1.25 | 0.50-1.00 | 0.25-0.50 | 0.25 |
|---|---|---|---|---|---|---|---|
| Fresh Weight (g) | - | - | - | - | 5.100 | 41.711 | 28.175 |
| Dry Weight (g) | - | - | - | - | 0.115 | 1.202 | 0.971 |
| Viability (%) | - | - | - | - | 6.900 | 6.200 | 2.600 |
| % Total Dry Weight | - | - | - | - | 5.000 | 52.500 | 42.500 |
| Viable Dry Weight | - | - | - | - | 0.019 | 0.074 | 0.025 |
| % Viable Total Weight | - | - | - | - | 16.100 | 62.700 | 21.200 |

## TABLE 10

Homogenate after 1 h, at 3100 RPM

| Size range (mm) | 3.0 | 0.75-3.00 | 1.25-2.75 | 1.00-1.25 | 0.50-1.00 | 0.25-0.50 | 0.25 |
|---|---|---|---|---|---|---|---|
| Fresh Weight (g) | - | - | 0.906 | 3.692 | 32.147 | 21.456 | 30.664 |
| Dry Weight (g) | - | - | 0.308 | 0.072 | 0.770 | 0.458 | 0.598 |
| Viability (%) | - | - | 15.000 | 42.700 | 58.900 | 27.200 | 12.500 |
| % Total Dry Weight | - | - | 14.000 | 3.300 | 34.900 | 20.800 | 27.000 |
| Viable Dry Weight | - | - | 1.046 | 0.031 | 0.435 | 0.125 | 0.075 |
| % Viable Total Weight | - | - | 6.300 | 4.200 | 62.000 | 17.10 | 10.400 |

0171970

## TABLE 11

Homogenate after 2 h, at 2,100 RPM

| Size range (mm) | 3.0 | 0.75 3.00 | 1.25 2.75 | 1.00 1.25 | 0.50- 1.00 | 0.25- 0.50 | 0.25 |
|---|---|---|---|---|---|---|---|
| Fresh Weight (g) | - | - | 0.457 | 6.298 | 27.934 | 16.539 | 4.886 |
| Dry Weight (g) | - | - | 0.014 | 0.239 | 0.802 | 0.495 | 0.812 |
| Viability (%) | - | - | 62.000 | 51.200 | 62.100 | 64.700 | 53.600 |
| % Total Dry Weight | - | - | 0.600 | 10.100 | 33.900 | 21.000 | 34.400 |
| Viable Dry Weight | - | - | 0.009 | 0.122 | 0.498 | 0.320 | 0.435 |
| % Viable Total Weight | - | - | 0.600 | 8.800 | 36.000 | 23.100 | 31.400 |

## TABLE 12

Loss of biomass during homogenisation. Final viable dry weight as percentage of initial viable dry weight.

| Speed RPM | 13,000 | 335 | 13,100 | 3,100 | 2,100 |
|---|---|---|---|---|---|
| Duration | 10 sec | 24 h | 17h | 1h | 2h |
| Final Viable Dry weight % of Initial | 24.9 | 10.1 | 8.9 | 30.5 | 47.4 |

CLAIMS

1. A method for the preparation of suspension cultures, which comprises exposing live plant tissue to mechanical shearing of sufficient intensity to form a suspension of particles of average size substantially smaller than the original tissue during a period sufficient to permit substantial comminution of the tissue without irreversible loss of viability of the suspension so formed and subsequently culturing the comminuted tissue in contact with a nutrient medium.

2. A method according to claim 1 wherein said live plant tissue is first mixed with a liquid medium.

3. A method according to claim 2 wherein said liquid medium is water or aqueous nutrient medium.

4. A method according to any foregoing claim wherein shear stress is applied by means of a rotary, reciprocating or ocillating member moving through the medium.

5. A method according to claim 4 wherein said member has a maximum speed greater than 3m $S^{-1}$

6. A method according to claim 5 wherein said maximum speed is from 5 to 45 m $s^{-1}$.

7. A method according to claim 4 wherein shear is imparted by a rotating member rotating at from 1,500 to 15,000 revolutions per minute.

8. A method according to claim 7 wherein the rotating member rotates at between 12,000 and 14,000 revolutions per minute.

9. A method according to any foregoing claim wherein the shear is applied for less than 2 hours.

10. A method according to claim 9 wherein the shear is applied for a period of from 2 seconds to 10 minutes.

11. A method according to claim 10 wherein the shear is applied for from 5 seconds to 5 minutes.

12. A method according to any foregoing claim wherein the supernatant liquid is separated from the comminuted cells after shearing.

13. A method according to claim 12 wherein the separation is effected by allowing the cells to sediment and decanting off the supernatant liquid.

14. A method according to any foregoing claim wherein the tissue is comminuted to a particle size less than 3mm.

15. A method according to claim 14 wherein substantially all the tissue comminuted is reduced to a particle size less than 1mm.

16. A method according to claim 15 wherein at least the major proportion by weight of the tissue comminuated is reduced to a particle size of less than 0.5mm.

17. A method according to any foregoing claim wherein the percentage viability of cells in the culture is maintained above 30% during shearing.

18. A method according to claim 15 wherein the percentage of viable cells in the culture is maintained above 60% during shearing.

19. A method according to any foregoing claim wherein at least a substantial proportion of the tissue is reduced to particles of less than 0.5mm and having a viability of at least 50% on cessation of shearing.

20. A method according to any foregoing claim for the comminution of live whole plants or pieces of tissue obtained directly there from.

21. A method according to any of claims 1 - 19 for comminuting a coarse suspension culture.

22. A method according to any of claims 1 - 19 for preparing a suspension culture from a callus tissue culture.

23. A method according to any foregoing claim wherein the suspension culture produced is contacted with open porous or reticular bodies having an internal voidage sufficient to permit invasion by, and immobiliiation or entrapment of, plant cells from the suspension.

24. A method according to any one of claims 1 - 22 wherein said suspension culture is used to inoculate a solid or gelatinous nutrient sustrate to form a callus tissue culture.

25. A method according to any foregoing claim sustantially as described herein with reference to any one of the examples.

26. A suspension culture prepared by the method of any foregoing claim.

27. An open porous or reticular body containing immobilised plant tissue formed by invasion of said body by cells from a suspension culture according to claim 26.

FIG.1

FIG.3

FIG.2